(19) Europäisches Patentamt
European Patent Office
Office européen des brevets

(11) EP 4 287 336 A1

(12) EUROPEAN PATENT APPLICATION

(43) Date of publication:
**06.12.2023 Bulletin 2023/49**

(21) Application number: **23176952.2**

(22) Date of filing: **02.06.2023**

(51) International Patent Classification (IPC):
***H01M 10/056*** *(2010.01)*

(52) Cooperative Patent Classification (CPC):
**H01M 10/056;** H01M 2300/0017

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.06.2022 KR 20220068034**

(71) Applicants:
- **SK Innovation Co., Ltd.**
  **Seoul 03188 (KR)**
- **SK On Co., Ltd.**
  **Seoul 03161 (KR)**

(72) Inventors:
- **SHIM, You Jin**
  **34124 Daejeon (KR)**
- **SHIM, Yu Na**
  **34124 Daejeon (KR)**
- **LEE, Min Young**
  **34124 Daejeon (KR)**

(74) Representative: **Bird & Bird LLP**
**Maximiliansplatz 22**
**80333 München (DE)**

# (54) ELECTROLYTE SOLUTION FOR LITHIUM SECONDARY BATTERY AND LITHIUM SECONDARY BATTERY INCLUDING THE SAME

(57) According to the present disclosures, an electrolyte solution for a lithium secondary battery and a lithium secondary battery including the electrolyte solution are provided. The electrolyte solution includes an additive represented by a specific chemical formula, an organic solvent and a lithium salt. The lithium secondary battery including the electrolyte solution provide enhanced high-temperature properties.

FIG. 1

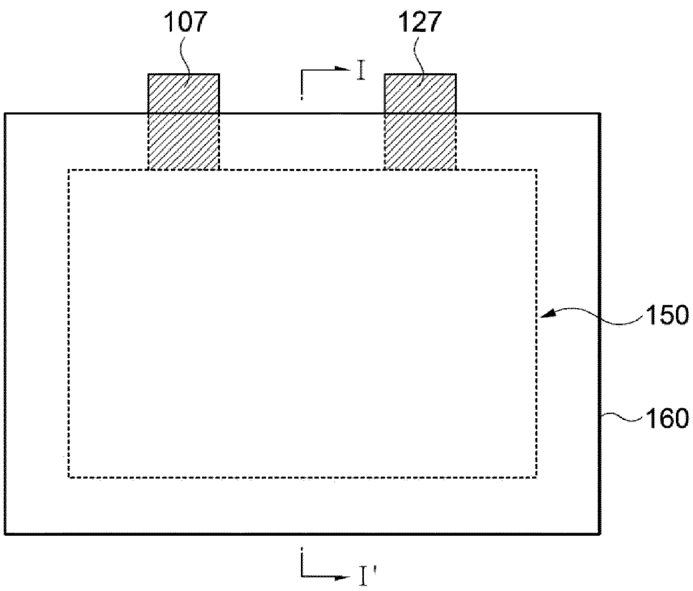


EP 4 287 336 A1

## Description

CROSS-REFERENCE TO RELATED APPLICATION AND CLAIM OF PRIORITY

This application claims priority to Korean Patent Applications No. 10- 2022-0068034 filed on June 3, 2022 in the Korean Intellectual Property Office (KIPO), the entire disclosure of which is incorporated by reference herein.

BACKGROUND

1. Field

The present disclosure of this patent document relates to an electrolyte solution and a lithium secondary battery including the same. More particularly, the disclosures of this patent document relate to an electrolyte solution including a solvent and an electrolytic salt, and a lithium secondary battery including the same.

2. Description of the Related Art

A secondary battery which can be charged and discharged repeatedly has been widely employed as a power source of a mobile electronic device such as a camcorder, a mobile phone, a laptop computer, etc.

A lithium secondary battery is actively developed and applied among various types of secondary batteries due to high operational voltage and energy density per unit weight, a high charging rate, a compact dimension, etc.

For example, the lithium secondary battery may include an electrode assembly including a cathode, an anode and a separation layer (separator), and an electrolyte solution immersing the electrode assembly.

A lithium metal oxide may be used as an active material for a cathode of the lithium secondary battery. Examples of the lithium metal oxide include a nickel-based lithium metal oxide.

As an application range of the lithium secondary batteries is expanded, enhanced life-span, and higher capacity and operational stability are required. Accordingly, a lithium secondary battery that provides uniform power and capacity even during repeated charging and discharging is preferable.

However, power and capacity may be decreased due to surface damages of a nickel-based lithium metal oxide used as the cathode active material, and side reactions between the nickel-based lithium metal oxide and the electrolyte may occur. Particularly, the lithium secondary battery may be placed in a high-temperature environment during repeated charging/discharging and overcharging. In this case, the above-described problems are accelerated, causing a battery expansion (gas generation at an inside of the battery, increase of a battery thickness), an increase of an internal resistance of the battery, and deterioration of life-span properties of the battery.

SUMMARY

According to an aspect of the present disclosures, there is provided an electrolyte solution providing improved high-temperature property and battery performance.

According to an aspect of the present disclosures, there is provided a lithium secondary battery having improved high--temperature property and battery performance.

An electrolyte solution for a lithium secondar battery includes an additive containing a compound represented by Chemical Formula 1, an organic solvent, and a lithium salt.

[Chemical Formula 1]

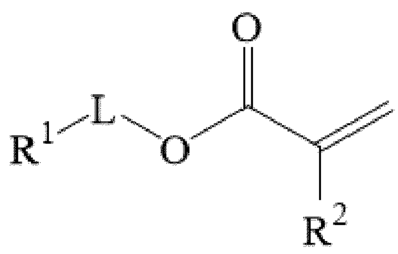

In Chemical Formula 1, $R^1$ is a substituted or unsubstituted $C_2$-$C_8$ cyclic ether group, L is a substituted or

unsubstituted $C_1$-$C_5$ alkylene group, and $R_2$ is hydrogen or a substituted or unsubstituted $C_1$-$C_5$ alkyl group.

In some embodiments, $R^1$ may be an unsubstituted $C_2$-$C_6$ cyclic ether group, L may be an unsubstituted $C_1$-$C_3$ alkylene group, and $R^2$ may be hydrogen or an unsubstituted $C_1$-$C_3$ alkyl group.

In some embodiments, $R^1$ may be an unsubstituted $C_2$-$C_4$ cyclic ether group, L may be an unsubstituted $C_1$ alkylene group, and $R^2$ may be hydrogen or an unsubstituted $C_1$ alkyl group.

In some embodiments, the additive may be included in an amount from 0.1 wt% to 5 wt% based on a total weight of the electrolyte solution.

In some embodiments, the additive may be included in an amount from 0.5 wt% to 2 wt% based on a total weight of the electrolyte solution.

In some embodiments, the organic solvent may include a linear carbonate-based solvent and a cyclic carbonate-based solvent.

In some embodiments, the electrolyte solution may further include at least one auxiliary additive selected from the group consisting of a cyclic carbonate-based compound, a fluorine-substituted carbonate-based compound, a sultone-based compound, a cyclic sulfate-based compound and a phosphate-based compound.

In some embodiments, the auxiliary additive may be included in an amount from 0.05 wt% to 10 wt% based on a total weight of the electrolyte solution.

In some embodiments, the auxiliary additive may be included in an amount from 0.1 wt% to 5 wt% based on a total weight of the electrolyte solution.

In some embodiments, a weight ratio of the auxiliary additive to the additive may be in a range from 0.1 to 10 in the electrolyte solution.

A lithium secondary battery includes an electrode assembly including a plurality of cathodes and a plurality of anodes which are repeatedly stacked, a case accommodating the electrode assembly, and the electrolyte solution for a lithium secondary battery of the above-described embodiments accommodated together with the electrode assembly in the case.

An electrolyte solution including an additive according to example embodiments may form a robust solid electrolyte interphase (SEI) on an electrode surface. Accordingly, a lithium secondary battery having improved high-temperature storage properties (e.g., improvement of a capacity retention, prevention of a resistance increase and a thickness increase at high temperature) may be implemented. Further, a lithium secondary battery having various enhanced battery performances (e.g., reduction of an initial resistance, improvement of rapid charge life-span capacity retention, improvement in a low-temperature capacity and life-span capacity retention at 25 °C) may also be implemented.

## BRIEF DESCRIPTION OF THE DRAWINGS

FIGS. 1 and 2 are a schematic plan view and a schematic cross-sectional view, respectively, illustrating a lithium secondary battery in accordance with exemplary embodiments.

## DETAILED DESCRIPTION OF THE EMBODIMENTS

In the present specification, the term "A-based compound" may refer to a compound including a group A and a derivative of the compound.

In the present specification, the term "Ca-Cb" refers that the number of carbon (C) atoms is from a to b.

### <Electrolyte Solution for Lithium Secondary Battery>

An electrolyte solution for a lithium secondary battery according to embodiments of the present disclosures includes a lithium salt, organic solvents and an additive including a compound represented by Chemical Formula 1, and may provide improved high-temperature properties and battery performance.

### Additive

The electrolyte solution for a lithium secondary battery according to exemplary embodiments may include an additive including a compound represented by Chemical Formula 1.

[Chemical Formula 1]

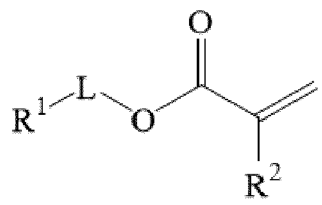

In Chemical Formula 1, for example, $R^1$ may be a substituted or unsubstituted $C_2$-C8 cyclic ether group. Preferably, $R^1$ may be an unsubstituted $C_2$-$C_6$ cyclic ether group, more preferably an unsubstituted $C_2$-$C_4$ cyclic ether group.

For example, $R^2$ may be hydrogen or a substituted or unsubstituted $C_1$-$C_5$ alkyl group. Preferably, $R^2$ may be hydrogen or an unsubstituted $C_1$-$C_3$ alkyl group, more preferably hydrogen or an unsubstituted $C_1$ alkyl group.

For example, L may be a substituted or unsubstituted $C_1$-$C_5$ alkylene group. Preferably L may be an unsubstituted $C_1$-$C_3$ alkylene group, more preferably an unsubstituted $C_1$-alkylene group.

For example, the cyclic ether group may refer to a heterocyclic compound including an ether bond in a cycloalkyl ring.

For example, the alkyl group may mean a partial remaining structure assuming that one hydrogen atom is removed from the alkane ($C_nH_{2n+2}$). For example, $CH_3$-$CH_2$-$CH_2$- indicates a propyl group.

For example, the alkylene group may refer to a structure in which one hydrogen atom is separated from each of carbon atoms at both terminals of an alkane. For example, -$CH_2$-$CH_2$-$CH_2$- indicates a propylene group.

For example, the ether may refer to a compound in which two carbon atoms are bonded to the same oxygen atom. For example, $CH_3$-O-$CH_3$ indicates dimethyl ether.

For example, the term "substituted" used herein refers that a substituent may be further bonded to a carbon atom of the alkylene group by substituting a hydrogen atom of the alkylene group with the substituent. For example, the substituent may be at least one of a halogen, a $C_1$-$C_6$ alkyl group, a $C_2$-$C_6$ alkenyl group, an amino group, a $C_1$-$C_6$ alkoxy group, a $C_3$-$C_7$ cycloalkyl group, and a 5 to 7-membered hetero-cycloalkyl group. In some embodiments, the substituent may be a halogen or a $C_1$-$C_6$ alkyl group.

When the additive containing the compound represented by Chemical Formula 1 is included in the electrolyte solution for a secondary battery, a film having reduced resistance may be formed on an electrode through, e.g., a decomposition reaction of the cyclic ether group. In one embodiment, an SEI film may be formed on an anode.

Therefore, decomposition of the organic solvent may be effectively prevented, and a gas generation and an increase of a battery thickness may be significantly reduced during high-temperature storage. Further, the film with relatively reduced resistance may be formed to prevent an increase of an initial resistance. Accordingly, mobility of lithium ions may be enhanced to remarkably improve rapid charging performance.

Therefore, high-temperature storage properties, room-temperature properties and initial resistance properties of the secondary battery may be improved together.

In one embodiment, the compound represented by Chemical Formula 1 may include (tetrahydrofuran-2-yl)methyl acrylate. For example, (tetrahydrofuran-2-yl)methyl acrylate is represented by Chemical Formula 1-1.

[Chemical Formula 1-1]

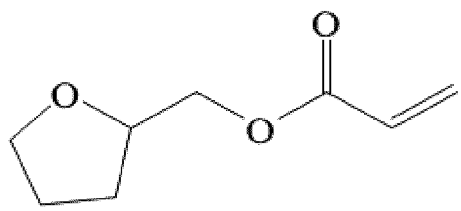

In one embodiment, the compound represented by Chemical Formula 1 may include (tetrahydrofuran-2-yl)methyl methacrylate. For example, (tetrahydrofuran-2-yl)methyl methacrylate is represented by Chemical Formula 1-2.

[Formula 1-2]

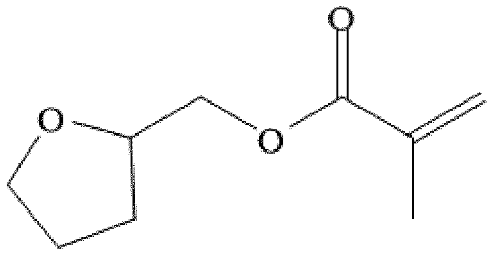

In one embodiment, the compound represented by Chemical Formula 1 may include glycidyl acrylate. Glycidyl acrylate is represented by Formula 1-3.

[Formula 1-3]

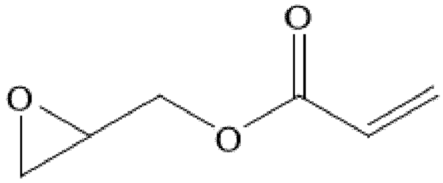

In example embodiments, the compound represented by Chemical Formulas 1-1 to 1-3 may be included as the additive of the electrolyte solution additive for a secondary battery, so that the stable SEI film may be formed on the anode by the decomposition reaction of tetrahydrofuran (THF) or an epoxy. Thus, the lithium secondary battery having improved high-temperature storage properties may be implemented. Additionally, decomposition of the electrolyte due to a reaction between the electrolyte and the anode may be suppressed, thereby reducing the gas generation.

Particularly, when compared to conventional additives providing high temperature storage properties, other battery performances such as an initial resistance, rapid charge properties, a room temperature life-span and low temperature properties may also be improved using the above-described additive.

In one embodiment, in a consideration of implementation of sufficient electrode passivation and stable SEI film formation, a content of the additive may be adjusted in a range from 0.1 weight percent (wt%) or more, 0.2 wt% or more, 0.3 wt% or more, 0.4 wt% or more, 0.5 wt% or more, or 1 wt% or more based on a total weight of the electrolyte solution. In consideration of the mobility of the lithium ions in the electrolyte solution and an active material activity, the content of the additive may be adjusted to 10 wt% or less, 9 wt% or less, 7 wt% or less, 6 wt% or less, 5 wt% or less, 4.5 wt% or less, 4 wt% or less, 3.5 wt% or less, 3 wt% or less, 2 wt% or less based on the total weight of the electrolyte solution.

In one embodiment, the content of the additive may be in a range from 0.1 wt% to 5 wt%, or 0.5 wt% to 2 wt%. Within the above range, the mobility of lithium ions and the activity of a cathode active material may be maintained while sufficiently implementing the passivation of the anode as described above. Further, improved capacity retention may be achieved at high temperature, increase of the resistance and the battery thickness may be suppressed more effectively.

Auxiliary Additive

The electrolyte solution for a rechargeable lithium battery according to example embodiments may further include an auxiliary additive together with the above additive. Durability and stability of the electrode may be further improved by the addition of the auxiliary additive. The auxiliary additive may be included in an appropriate amount within a range that may not inhibit the mobility of lithium ions in the electrolyte.

The auxiliary additive may include, e.g., a cyclic carbonate-based compound, a fluorine-substituted carbonate-based compound, a sultone-based compound, a cyclic sulfate-based compound and a phosphate-based compound.

In some embodiments, a content of the auxiliary additive may be adjusted in a range from 10 wt% or less, 9 wt% or less, 8 wt% or less, 7 wt% or less, 6 wt% or less, 5wt% or less, 4 wt% or less, 3 wt% or less, 2 wt% or less, or 1 wt% or less based on the total weight of the electrolyte solution in consideration of an interaction with the additive including the compound represented by Chemical Formula 1

In an embodiment, the auxiliary additive may be included in an amount from about 0.05 wt% to 10 wt, or 0.1

wt% to 5 wt% based on the total weight of the electrolyte solution. Within the above range, durability of the electrode protective film may be improved, and improvements of high-temperature storage properties and battery performances may be easily implemented without degrading the function of the additive.

In an embodiment, a weight ratio of the auxiliary additive relative to a weight of the additive in the electrolyte solution may be from 0.1 to 10, more than 1 and 10 or less, or from 5 to 10. In this case, the lithium secondary battery having improved cycle properties and the high-temperature storage properties may be implemented due to the interaction between the additive and the auxiliary additive.

The cyclic carbonate-based compound may include vinylene carbonate (VC), vinyl ethylene carbonate (VEC), etc.

The fluorine-substituted cyclic carbonate-based compound may include fluoroethylene carbonate (FEC).

The sultone-based compound may include 1,3-propane sultone, 1,3-propene sultone, 1,4-butane sultone. etc.

The cyclic sulfate-based compound may include 1,2-ethylene sulfate, 1,2-propylene sulfate, etc.

The phosphate-based compound may include an oxalatophosphate-based compound such as lithium bis(oxalato)phosphate.

In an embodiment, the fluorine-substituted cyclic carbonate-based compound, the sultone-based compound, the cyclic sulfate-based compound and the oxalatophosphate-based compounds may be used together as the auxiliary additive.

Organic Solvent and Lithium Salt

The organic solvent may include an organic compound that provides sufficient solubility for the lithium salt, the additive and the auxiliary additive and may have no substantial reactivity in the lithium secondary battery.

For example, the organic solvent may include a carbonate-based solvent, an ester-based solvent, an ether-based solvent, a ketone-based solvent, an alcohol-based solvent, an aprotic solvent, etc. These may be used alone or in a combination thereof.

In some embodiments, the organic solvent may include a carbonate-based solvent. The carbonate-based solvent may include a linear carbonate-based solvent and a cyclic carbonate-based solvent.

For example, the linear carbonate-based solvent may include at least one of dimethyl carbonate (DMC), ethyl methyl carbonate (EMC), diethyl carbonate (DEC), methyl propyl carbonate, ethyl propyl carbonate and dipropyl carbonate.

For example, the cyclic carbonate-based solvent may include at least one of ethylene carbonate (EC), propylene carbonate (PC) and butylene carbonate.

In some embodiments, in the organic solvent, an amount of the linear carbonate-based solvent is greater than an amount of the cyclic carbonate-based solvent based on a volume.

For example, a mixed volume ratio of the linear carbonate-based solvent and the cyclic carbonate-based solvent may be from 1:1 to 9:1, and may be from 1.5:1 to 4:1 in an embodiment.

For example, the ester-based solvent may include at least one of methyl acetate (MA), ethyl acetate (EA), n-propyl acetate (n-PA), 1,1-dimethylethyl acetate (DMEA), methyl propionate (MP) and ethyl propionate (EP).

For example, the ether-based solvent may include at least one of dibutyl ether, tetraethylene glycol dimethyl ether (TEGDME), diethylene glycol dimethyl ether (DEGDME), dimethoxyethane, tetrahydrofuran (THF) and 2-methyltetrahydrofuran.

For example, the ketone-based solvent may include cyclohexanone. The alcohol-based solvent may include at least one of ethyl alcohol and isopropyl alcohol.

For example, the aprotic solvent may include at least one of a nitrile-based solvent, an amide-based solvent (e.g., dimethylformamide), a dioxolane-based solvent (e.g., 1,3-dioxolane) and a sulfolane-based solvent.

The electrolyte may include, e.g., a lithium salt. For example, the lithium salt may be expressed as $Li^+X^-$. Non-limiting examples of the anion $X^-$ may include $F^-$, $Cl^-$, $Br^-$, $I^-$, $NO_3^-$, $N(CN)_2^-$, $BF_4^-$, $ClO_4^-$, $PF_6^-$, $(CF_3)_2PF_4^-$, $(CF_3)_3PF_3^-$, $(CF_3)_4PF_2^-$, $(CF_3)_5PF^-$, $(CF_3)_6P^-$, $CF_3SO_3^-$, $CF_3CF_2SO_3^-$, $(CF_3SO_2)_2N^-$, $(FSO_2)_2N^-$, $CF_3CF_2(CF_3)_2CO^-$, $(CF_3SO_2)_2CH^-$, $(SF_5)_3C^-$, $(CF_3SO_2)_3C^-$, $CF_3(CF_2)_7SO_3^-$, $CF_3CO_2^-$, $CH_3CO_2^-$, $SCN^-$ , $(CF_3CF_2SO_2)_2N^-$, etc.

In some embodiments, the lithium salt may include at least one of $LiBF_4$ and $LiPF_6$.

In an embodiment, the lithium salt may be included in a concentration from about 0.01 M to 5M, or from 0.01 M to 2M with respect to the organic solvent. Within the above range, transfer of lithium ions and/or electrons may be promoted during charging and discharging of the lithium secondary battery.

<Lithium Secondary Battery>

FIGS. 1 and 2 are a schematic plan view and a schematic cross-sectional view, respectively, illustrating a lithium secondary battery in accordance with exemplary embodiments. FIG. 2 is cross-sectional view taken along a line

I-I' of FIG. 1.

Referring to FIGS. 1 and 2, a lithium secondary battery may include an electrode assembly including a cathode 100 and an anode 130 facing the cathode 100.

The cathode 100 may include a cathode current collector 105 and a cathode active material layer 110 on the cathode current collector 105. The cathode active material layer 110 may include a cathode active material. The cathode active material layer 110 may further include a cathode binder and a conductive material.

For example, a cathode slurry may be prepared by mixing and stirring the cathode active material, the cathode binder, the conductive material, a dispersive medium, etc., and then the cathode slurry may be coated on the cathode current collector 105, dried and pressed to form the cathode 100.

For example, the cathode current collector 105 may include stainless steel, nickel, aluminum, titanium, copper, or an alloy thereof.

The cathode active material may include lithium metal oxide particles capable of reversibly intercalating and de-intercalating lithium ions. In an embodiment, the cathode active material may include the lithium metal oxide particles containing nickel.

In some embodiments, the lithium metal oxide particle may include 80 mol% or more of nickel based on the total number of moles of all elements except lithium and oxygen. In this case, the lithium secondary battery having a high capacity may be implemented.

In some embodiments, the lithium metal oxide particle may include 83 mol% or more, 85 mol% or more, 90 mol% or more, or 95 mol% or more of nickel based on the total number of moles of all elements except lithium and oxygen.

In some embodiments, the lithium metal oxide particle may further include at least one of cobalt and manganese.

In some embodiments, the lithium metal oxide particle may further include cobalt and manganese. In this case, the lithium secondary battery having enhanced power and penetration stability may be implemented.

In one embodiment, the lithium metal oxide particle may be represented by Chemical Formula 2 below.

[Chemical Formula 2] $Li_xNi_{(1-a-b)}Co_aM_bO_y$

For example, in Chemical Formula 2, M may include at least one of Al, Zr, Ti, Cr, B, Mg, Mn, Ba, Si, Y, W and Sr, $0.9 \le x \le 1.2$, $1.9 \le y \le 2.1$, and $0 \le a+b \le 0.5$.

In some embodiments, $0<a+b \le 0.4$, $0<a+b \le 0.3$, $0<a+b \le 0.2$, $0<a+b \le 0.17$, $0<a+b \le 0.15$, $0<a+b \le 0.12$, and $0<a+b \le 0.1$.

In an embodiment, the lithium metal oxide particles may further include a coating element or a doping element. For example, the coating element or doping element may include Al, Ti, Ba, Zr, Si, B, Mg, P, Sr, W, La, an alloy thereof, or and oxide thereof. In this case, the lithium secondary battery having improved life-span properties may be implemented.

For example, the cathode binder may include an organic based binder such as polyvinylidenefluoride (PVDF), a polyvinylidene fluoride-hexafluoropropylene copolymer (PVDF-co-HFP), polyacrylonitrile, polymethylmethacrylate, etc., or an aqueous based binder such as styrene-butadiene rubber (SBR) that may be used with a thickener such as carboxymethyl cellulose (CMC).

For example, the conductive material may include a carbon-based material such as graphite, carbon black, graphene, carbon nanotube, etc., and/or a metal-based material such as tin, tin oxide, titanium oxide, a perovskite material such as $LaSrCoO_3$ or $LaSrMnO_3$, etc.

The anode 130 may include an anode current collector 125 and an anode active material layer 120 on the anode current collector 125. The anode active material layer 120 may include an anode active material, and may further include an anode binder and a conductive material.

For example, the anode active material may be mixed and stirred together with a binder and conductive material, etc., in a solvent to form an anode slurry. The anode slurry may be coated on the anode current collector 125, dried and pressed to obtain the anode 130.

For example, the anode current collector 125 may include gold, stainless steel, nickel, aluminum, titanium, copper, or an alloy thereof. In one embodiment, the anode current collector 125 may include copper or a copper alloy.

The anode active material may be a material capable of intercalating and de-intercalating lithium ions. For example, the anode active material may include a lithium alloy, a carbon-based material, a silicon-based material, etc.

For example, the lithium alloy may include a metal element such as aluminum, zinc, bismuth, cadmium, antimony, silicon, lead, tin, gallium, indium, etc.

For example, the carbon-based material may include a crystalline carbon, an amorphous carbon, a carbon composite, a carbon fiber, etc.

For example, the amorphous carbon may include hard carbon, coke, a mesocarbon microbead (MCMB) calcined at 1500 °C or less, a mesophase pitch-based carbon fiber (MPCF), etc. The crystalline carbon may include, e.g., natural graphite, graphitized coke, graphitized MCMB, graphitized MPCF, etc.

In one embodiment, the anode active material may include the silicon-based material. For example, the silicon-based material may include Si, SiOx (0<x<2), Si/C, SiO/C, a Si-Metal, etc. In this case, a lithium secondary battery having a high capacity may be implemented.

For example, when the anode active material includes the silicon-based material, the battery thickness may be increased during repeated charging and discharging. The lithium secondary battery according to embodiments of the present disclosures may include the above-described electrolyte solution, so that the increase of the battery thickness may be reduced or suppressed.

In some embodiments, a content of the silicon-based active material in the anode active material may be in a range from 1 wt% to 20 wt%, 1 wt% to 15 wt%, or 1 wt% to 10 wt%.

The anode binder and the conductive material may include materials substantially the same as or similar the above-described cathode binder and conductive material. For example, the anode binder may include the aqueous binder such as styrene-butadiene rubber (SBR) that may be used together with a thickener such as carboxymethyl cellulose (CMC).

In some embodiments, a separation layer 140 may be interposed between the cathode 100 and the anode 130.

In some embodiments, an area of the anode 130 may be greater than an area of the cathode 100. In this case, lithium ions generated from the cathode 100 may be easily transferred to the anode 130 without being precipitated.

The separation layer 140 may include a porous polymer film prepared from, e.g., a polyolefin-based polymer such as an ethylene homopolymer, a propylene homopolymer, an ethylene/butene copolymer, an ethylene/hexene copolymer, an ethylene/methacrylate copolymer, or the like. The separation layer 140 may also include a non-woven fabric formed from a glass fiber with a high melting point, a polyethylene terephthalate fiber, or the like.

In example embodiments, an electrode cell may be defined by the cathode 100, the anode 130 and the separation layer 140, and a plurality of the electrode cells may be stacked to form an electrode assembly 150. For example, the electrode assembly 150 may be formed by winding, laminating or z-folding of the separation layer 140.

The lithium secondary battery according to example embodiments may include a cathode lead 107 connected to the cathode 100 to protrude to an outside of a case 160, and an anode lead 127 connected to the anode 130 to protrude to the outside of the case 160.

For example, the cathode lead 107 may be electrically connected to the cathode current collector 105. The anode lead 127 may be electrically connected to the anode current collector 125.

The cathode current collector 105 may include a protrusion (a cathode tab, not illustrated) at one side thereof. The cathode active material layer 110 may not be formed on the cathode tab. The cathode tab 106 may be integral with the cathode current collector 105 or may be connected to the cathode current collector 105 by, e.g., welding. The cathode current collector 105 and the cathode lead 107 may be electrically connected via the cathode tab.

The anode current collector 125 may include a protrusion (an anode tab, not illustrated) at one side thereof. The anode active material layer 120 may not be formed on the anode tab. The anode tab 126 may be integral with the anode current collector 125 or may be connected to the anode current collector 125 by, e.g., welding. The anode electrode current collector 125 and the anode lead 127 may be electrically connected via the anode tab.

The electrode assembly 150 may include a plurality of the cathodes and a plurality of the anodes. For example, the cathodes and the anodes may be alternately disposed, and the separation layer may be interposed between the cathode and the anode. Each of the plurality of the cathodes may include the cathode tab. Each of the plurality of the anodes may include the anode tab.

In an embodiment, the cathode tabs (or the anode tabs) may be laminated, pressed and welded to form a cathode tab stack (or an anode tab stack). The cathode tab stack may be electrically connected to the cathode lead 107. The anode tab stack may be electrically connected to the anode lead 127.

The electrode assembly 150 may be accommodated together with the electrolyte solution according to the above-described embodiments in a case 160 to form the lithium secondary battery.

The lithium secondary battery may be fabricated into a cylindrical shape using a can, a prismatic shape, a pouch shape, a coin shape, etc.

Hereinafter, specific examples and comparative examples are proposed to more concretely describe the present invention. However, the following examples are only given for illustrating the present invention and those skilled in the related art will obviously understand that various alterations and modifications are possible within the scope and spirit of the present invention. Such alterations and modifications are duly included in the appended claims.

Examples and Comparative Examples

(1) Synthesis of (tetrahydrofuran-2-yl)methyl acrylate (THFA)

After adding dichloromethane (30mL), tetrahydrofurfuryl alcohol (3.0g, 29mmol), and triethylamine (2.97g, 29mmol) to a 100mL round bottom flask, the flask was cooled to 0°C using ice water.

Acryloyl chloride (2.66 g, 29 mmol) was slowly added dropwise to the cooled product and stirred at room

temperature for 3 hours or more. The obtained product was slowly added dropwise to distilled water, and an organic solvent in a lower layer was separated from an aqueous solution.

The separated organic solvent layer was washed 2-3 times with an aqueous sodium chloride solution, and the solvent was removed under reduced pressure. The concentrated liquid was purified by a column chromatography using silica to obtain 3.49 g of (tetrahydrofuran-2-yl)methyl acrylate. (yield: 76%)

$^1H$ NMR (500MHz, $CDCl_3$): δ 6.4 (1H, d), 6.2 (1H, d), 5.9 (1H, s), 4.2 (3H, m), 3.9 (2H, m), 1.9 (3H, m), 1.6 (1H, m)

(2) Synthesis of (tetrahydrofuran-2-yl)methyl methacrylate (THFMA)

After adding dichloromethane (30mL), tetrahydrofurfuryl alcohol (3.0g, 29mmol) and triethylamine (2.97g, 29mmol) to a 100mL round bottom flask, the flask was cooled to 0°C using ice water.

To the cooled product, methacryloyl chloride (3.07 g, 29 mmol) was slowly added dropwise and stirred at room temperature for 3 hours or more. The product was slowly added dropwise to distilled water, and an organic solvent in a lower layer was separated from an aqueous solution.

The separated organic solvent layer was washed 2-3 times with an aqueous sodium chloride solution, and the solvent was removed under reduced pressure. The concentrated liquid was purified by a column chromatography using silica to obtain 3.05 g of (tetrahydrofuran-2-yl)methyl methacrylate (yield: 61%).

$^1H$ NMR (500MHz, $CDCl_3$): δ 6.2 (1H, s), 5.6 (1H, s), 4.2 (3H, m), 3.9 (2H, m), 2.0 (6H, m), 1.7 (1H, m)

(3) Synthesis of glycidyl acrylate (GA)

After adding dichloromethane (30mL), glycidol (3.0g, 40mmol) and triethylamine (4.1g, 40mmol) to a 100mL round bottom flask, the flask was cooled to 0°C using ice water.

Acryloyl chloride (3.67 g, 40 mmol) was slowly added dropwise to the cooled product and stirred at room temperature for 3 hours or more. The obtained product was slowly added dropwise to distilled water, and an organic solvent in a lower layer was separated from an aqueous solution.

The separated organic solvent layer was washed 2-3 times with an aqueous sodium chloride solution, and the solvent was removed under reduced pressure. The concentrated liquid was purified by a column chromatography using silica to obtain 2.85 g of glycidyl acrylate (yield: 55%).

$^1H$ NMR (500MHz, $CDCl_3$): δ 6.2 (1H, d), 5.9 (1H, d), 5.7 (1H, s), 4.2 (1H, d), 3.8 (1H, d), 3.0 (1H, t) , 2.7(1H, d), 2.4(1H, d)

(4) Preparation of Electrolyte Solution

A 1.0 M $LiPF_6$ solution (EC/EMC mixed solvent in a 25:75 volume ratio) was prepared.

In the $LiPF_6$ solution, the additives prepared in the above (1) to (3) and auxiliary additives were added and mixed with contents (wt%) shown in Table 1 below based on a total weight of an electrolyte solution to form electrolyte solutions of Examples and Comparative Examples.

(5) Fabrication of Lithium Secondary Battery Sample

$Li[Ni_{0.8}Co_{0.1}Mn_{0.1}]O_2$, carbon black and polyvinylidene fluoride (PVDF) were dispersed in N-methyl pyrrolidone (NMP) in a weight ratio of 98:1:1 to prepare a cathode slurry.

The cathode slurry was uniformly coated on an aluminum foil having a thickness of 12 μm, dried and pressed to prepare a cathode.

Anode active material including artificial graphite and natural graphite mixed in a weight ratio of 7:3, a conductive material, styrene-butadiene rubber (SBR) and carboxymethyl cellulose (CMC) were dispersed in water by a weight ratio of 96:3:1:1 to form an anode slurry. The anode slurry was uniformly coated on a copper foil having a thickness of 8 μm, dried and pressed to prepare an anode.

The prepared cathodes and anodes were alternately and repeatedly stacked, and a separator (13 μm-thickness, polyethylene film) was interposed between the cathode and the anode to prepare an electrode assembly.

The electrode assembly was inserted in a pouch, the electrolyte solution prepared in the above (4) was injected and sealed and impregnated for 12 hours to prepare a lithium secondary battery sample.

[Table 1]

| | Additive | Auxiliary Additive (wt%) | | |
|---|---|---|---|---|
| | | FEC | PS | PRS |
| Example 1 | THFA, 0.5 wt% | 1 | 0.5 | - |
| Example 2 | THFA, 1.0 wt% | 1 | 0.5 | - |
| Example 3 | THFA, 0.5 wt% | 1 | 0.5 | 0.5 |
| Example 4 | THFA, 1.0 wt% | 1 | 0.5 | 0.5 |
| Example 5 | THFMA, 0.5 wt% | 1 | 0.5 | - |
| Example 6 | GA, 0.5 wt% | 1 | 0.5 | - |
| Comparative Example 1 | - | 1 | 0.5 | 0.5 |
| Comparative Example 2 | - | 1 | 0.5 | - |

The components listed in Table 1 are as follows.

THFA: (tetrahydrofuran-2-yl)methyl acrylate
THFMA: (tetrahydrofuran-2-yl)methyl methacrylate
GA: glycidyl acrylate
FEC: fluoro-ethylene carbonate
PS: 1,3-propane sultone
PRS: prop-1-ene-1,3-sultone

Experimental Example

(1) High Temperature Storage Properties

(1-1) Measurement of capacity retention (Ret) after high temperature storage

The lithium secondary batteries of Examples and Comparative Examples were subjected to 0.5C CC/CV charging (4.2V, 0.05C CUT-OFF) and 0.5C CC discharging (2.7V CUT-OFF) at 25 °C by three cycles, and a discharge capacity C1 at the 3rd cycle was measured. After storing the charged lithium secondary battery at 60° C for 12 weeks, the batteries were additionally maintained at room temperature for 30 minutes, and a discharge capacity C2 was measured by 0.5C CC discharging (2.75V CUT-OFF). A capacity retention was calculated as follows.

$$\text{capacity retention (\%)} = C2/C1 \times 100\ (\%)$$

(1-2) Measurement of internal resistance (DCIR) increase ratio after high temperature storage

The lithium secondary battery of each Examples and Comparative Examples was 0.5C CC/CV charged (4.2V 0.05C CUT-OFF) at 25 °C, and then 0.5C CC discharged until a SOC 60%. At the SOC 60% point, DCIR R1 was measured by discharging for 10 seconds and complementary charging while changing the C-rate to 0.2C, 0.5C, 1C, 1.5C, 2C and 2.5C

The charged lithium secondary battery was exposed to air at 60 °C for 12 weeks. The battery was further left at room temperature for 30 minutes, and then DCIR R2 was measured by the same method as described above. An internal resistance increase ratio was calculated as follows.

$$\text{Internal resistance increase ratio (\%)} = (R2-R1)/R1 \times 100\ (\%)$$

(1-3) Measurement of battery thickness after high temperature storage

After charging the lithium secondary batteries of Examples and Comparative Examples at 25 °C under conditions of 0.5C CC/CV (4.2V 0.05C CUT-OFF), a battery thickness T1 was measured.

After exposing the charged lithium secondary batteries to air at 60 °C for 12 weeks (using a thermostat), a battery thickness T2 was measured. The battery thickness was measured using a plate thickness measuring device (Mitutoyo, 543-490B). A battery thickness increase ratio was calculated as follows.

$$\text{battery thickness increase ratio (\%)} = (T2\text{-}T1)/T1 \times 100\ (\%)$$

(2) Initial DCIR

The lithium secondary battery of each Examples and Comparative Examples was 0.5C CC/CV charged (4.2V 0.05C CUT-OFF) at 25 °C, and then 0.5C CC discharged until a SOC 60%. At the SOC 60% point, an initial DCIR was measured by discharging for 10 seconds and complementary charging while changing the C-rate to 0.2C, 0.5C, 1C, 1.5C, 2C and 2.5C.

(3) Rapid charge capacity retention

Lithium secondary batteries of Examples and Comparative Examples were charged at 0.33C to SOC 8%, and charged stepwise by 2.5C-2.25C-2C-1.75C-1.5C-1.0C in an SOC 8-80% section, charged again at 0.33C (4.2V, 0.05C cut-off) in an SOC 80-100% section, and then CC discharged to 2.7V at 0.33C.

A discharge capacity at the first cycle A1 was measured, and the charge and discharge were repeated 100 times to measure a discharge capacity A2 at the 100th cycle.

A rapid charge capacity retention was calculated as follows.

$$\text{rapid charge capacity retention (\%)} = A2/A1 \times 100\ (\%)$$

(4) Low temperature capacity

The lithium secondary batteries of Examples and Comparative Examples were 0.5C CC/CV charged (4.2V, 0.05C CUT-OFF) at 25°C, left at -10 °C for 4 hours, and then 0.5C CC discharged (2.7V CUT-OFF) to measure a discharge capacity (mAh).

(5) 25°C capacity retention

The lithium secondary batteries of Examples and Comparative Examples were subjected to 0.5C CC/CV charging (4.2V, 0.05C CUT-OFF) and 0.5C CC discharging (2.7V CUT-OFF) 500 times at 25°C. A capacity retention was measured by dividing a discharge capacity at the 500th cycle by a discharge capacity at the first cycle.

The evaluation results are shown in Tables 2 and 3 below.

[Table 2]

| | high temperature storage properties | | |
|---|---|---|---|
| | Ret.(%) | DCIR increase ratio (%) | thickness increase ratio (%) |
| Example 1 | 90.9 | 103.1 | 75.7 |
| Example 2 | 92.5 | 99.8 | 59.1 |
| Example 3 | 93.1 | 102.3 | 63.6 |
| Example 4 | 93.9 | 105.1 | 50.1 |
| Example 5 | 92.3 | 105.6 | 87.3 |
| Example 6 | 92.3 | 104.3 | 85.4 |

(continued)

| | high temperature storage properties | | |
|---|---|---|---|
| | Ret.(%) | DCIR increase ratio (%) | thickness increase ratio (%) |
| Comparative Example 1 | 88.2 | 108.6 | 96.2 |
| Comparative Example 2 | 86.1 | 115.5 | 116.4 |

[Table 3]

| | Battery Performance | | | |
|---|---|---|---|---|
| | initial DCIR (mΩ) | rapid charge capacity retention (%, 100cycles) | low temperature capacity (mAh) | 25°C capacity retention (%, 500cycles) |
| Example 1 | 36.2 | 92.9 | 1510 | 89.7 |
| Example 2 | 37.0 | 93.1 | 1505 | 90.2 |
| Example 3 | 40.4 | 92.1 | 1368 | 87.1 |
| Example 4 | 40.2 | 91.8 | 1360 | 87.0 |
| Example 5 | 40.4 | 91.9 | 1500 | 89.9 |
| Example 6 | 40.3 | 92.3 | 1501 | 90.0 |
| Comparative Example 1 | 40.5 | 90.3 | 1399 | 86.8 |
| Comparative Example 2 | 35.9 | 93.8 | 1525 | 90.8 |

Referring to Table 2, the lithium secondary batteries according to Examples provided improved results in high-temperature storage evaluation (capacity retention, resistance increase ratio and thickness increase ratio).

Referring to Table 3, the lithium secondary batteries according to Examples provided improved results in other battery performances (initial resistance, rapid charge capacity retention, low temperature capacity, room temperature capacity retention).

Referring to Comparative Examples 1 and 2, high-temperature properties were improved by including PRS, but other battery performances were deteriorated. However, in Examples, other battery performances were also improved together with the high temperature storage properties.

## Claims

**1.** An electrolyte solution for a lithium secondary battery, comprising:

an additive containing a compound represented by Chemical Formula 1;
an organic solvent; and
a lithium salt:

[Chemical Formula 1]

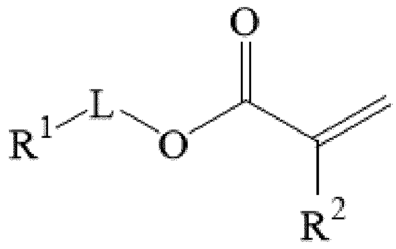

wherein, in Chemical Formula 1, $R^1$ is a substituted or unsubstituted $C_2$-$C_8$ cyclic ether group, L is a substituted or unsubstituted $C_1$-$C_5$ alkylene group, and $R_2$ is hydrogen or a substituted or unsubstituted $C_1$-$C_5$ alkyl group.

**2.** The electrolyte solution for a lithium secondary battery according to claim 1, wherein $R^1$ is an unsubstituted $C_2$-$C_6$ cyclic ether group, L is an unsubstituted $C_1$-$C_3$ alkylene group, and $R^2$ is hydrogen or an unsubstituted $C_1$-$C_3$ alkyl group.

**3.** The electrolyte solution for a lithium secondary battery according to claims 1 or 2, wherein $R^1$ is an unsubstituted $C_2$-$C_4$ cyclic ether group, L is an unsubstituted $C_1$ alkylene group, and $R^2$ is hydrogen or an unsubstituted $C_1$ alkyl group.

**4.** The electrolyte solution for a lithium secondary battery according to one of claims 1 to 3, wherein the additive is included in an amount from 0.1 wt% to 5 wt% based on a total weight of the electrolyte solution.

**5.** The electrolyte solution for a lithium secondary battery according to one of claims 1 to 4, wherein the additive is included in an amount from 0.5 wt% to 2 wt% based on a total weight of the electrolyte solution.

**6.** The electrolyte solution for a lithium secondary battery according to one of claims 1 to 5, wherein the organic solvent includes a linear carbonate-based solvent and a cyclic carbonate-based solvent.

**7.** The electrolyte solution for a lithium secondary battery according to one of claims 1 to 6, further comprising at least one auxiliary additive selected from the group consisting of a cyclic carbonate-based compound, a fluorine-substituted carbonate-based compound, a sultone-based compound, a cyclic sulfate-based compound and a phosphate-based compound.

**8.** The electrolyte solution for a lithium secondary battery according to claim 7, wherein the auxiliary additive is included in an amount from 0.05 wt% to 10 wt% based on a total weight of the electrolyte solution.

**9.** The electrolyte solution for a lithium secondary battery according to claims 7 or 8, wherein the auxiliary additive is included in an amount from 0.1 wt% to 5 wt% based on a total weight of the electrolyte solution.

**10.** The electrolyte solution for a lithium secondary battery according to one of claims 7 to 9, a weight ratio of the auxiliary additive to the additive is in a range from 0.1 to 10 in the electrolyte solution.

**11.** A lithium secondary battery, comprising:

an electrode assembly comprising a plurality of cathodes and a plurality of anodes which are repeatedly stacked;
a case accommodating the electrode assembly; and
the electrolyte solution for a lithium secondary battery of any one of claims 1 to 10 accommodated together with the electrode assembly in the case.

FIG. 1

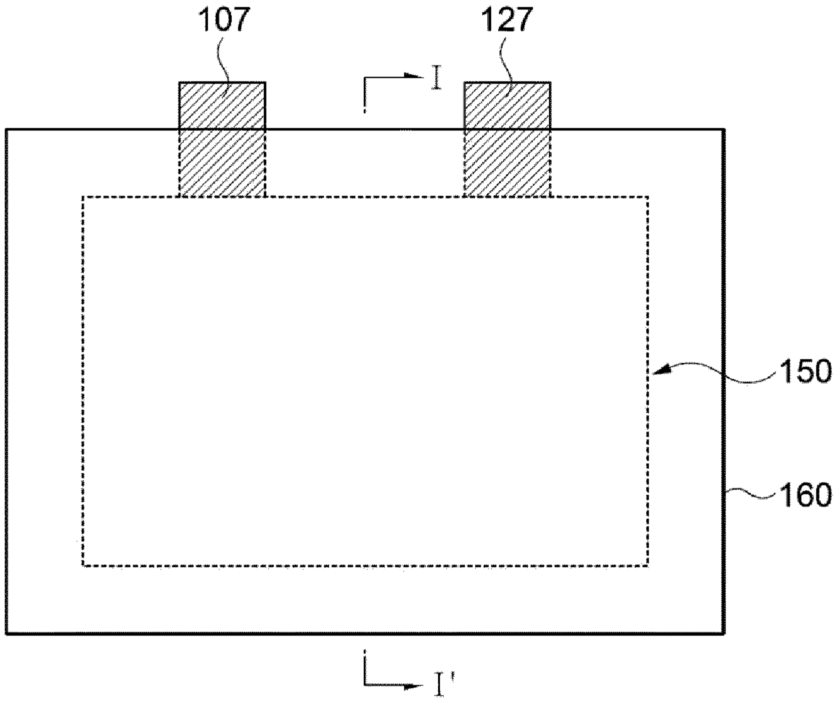


FIG. 2

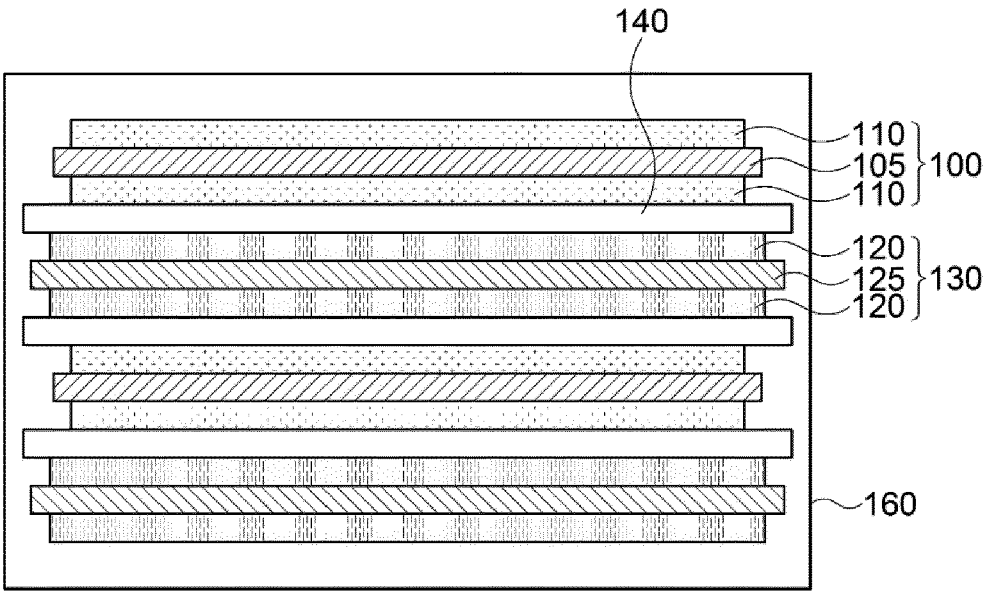

Europäisches Patentamt
European Patent Office
Office européen des brevets

# EUROPEAN SEARCH REPORT

Application Number
EP 23 17 6952

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/146786 A1 (SATO TAKAYA [JP] ET AL) 29 July 2004 (2004-07-29)<br>* example 2; table 1 *<br>* paragraph [0061]; compound A4 *<br>* the whole document *<br>----- | 1-11 | INV.<br>H01M10/056 |
| X | CN 103 474 697 B (AMPEREX TECHNOLOGY LTD) 7 September 2016 (2016-09-07)<br>* example 1 *<br>* the whole document *<br>----- | 1-11 | |
| X | WO 2013/176232 A1 (ZEON CORP [JP]) 28 November 2013 (2013-11-28)<br>* examples 1,2,5 *<br>* the whole document *<br>----- | 1-11 | |
| X | KR 2021 0146293 A (OSAKA SODA CO LTD [JP]) 3 December 2021 (2021-12-03)<br>* paragraph [0042]; claims 1-5 *<br>* the whole document *<br>----- | 1-11 | TECHNICAL FIELDS SEARCHED (IPC) |
| X | WO 2022/070951 A1 (FUJIFILM WAKO PURE CHEMICAL CORP [JP]; FUJIFILM CORP [JP]) 7 April 2022 (2022-04-07)<br>* 119, compound D-15; table 1 *<br>* the whole document *<br>----- | 1-11 | H01M |

The present search report has been drawn up for all claims

1

| Place of search | Date of completion of the search | Examiner |
|---|---|---|
| The Hague | 22 September 2023 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 17 6952

This annex lists the patent family members relating to the patent documents cited in the above-mentioned European search report. The members are as contained in the European Patent Office EDP file on
The European Patent Office is in no way liable for these particulars which are merely given for the purpose of information.

22-09-2023

| Patent document cited in search report | Publication date | Patent family member(s) | Publication date |
|---|---|---|---|
| US 2004146786 A1 | 29-07-2004 | CN 1507669 A | 23-06-2004 |
| | | EP 1403957 A1 | 31-03-2004 |
| | | JP WO2002093679 A1 | 02-09-2004 |
| | | KR 20040005954 A | 16-01-2004 |
| | | TW 561640 B | 11-11-2003 |
| | | US 2004146786 A1 | 29-07-2004 |
| | | WO 02093679 A1 | 21-11-2002 |
| CN 103474697 B | 07-09-2016 | CN 103474697 A | 25-12-2013 |
| | | JP 2015056402 A | 23-03-2015 |
| | | US 2015072244 A1 | 12-03-2015 |
| WO 2013176232 A1 | 28-11-2013 | CN 104221194 A | 17-12-2014 |
| | | JP 6168051 B2 | 26-07-2017 |
| | | JP WO2013176232 A1 | 14-01-2016 |
| | | KR 20150021018 A | 27-02-2015 |
| | | WO 2013176232 A1 | 28-11-2013 |
| KR 20210146293 A | 03-12-2021 | CN 114008830 A | 01-02-2022 |
| | | JP WO2020203664 A1 | 08-10-2020 |
| | | KR 20210146293 A | 03-12-2021 |
| | | WO 2020203664 A1 | 08-10-2020 |
| WO 2022070951 A1 | 07-04-2022 | CN 116018362 A | 25-04-2023 |
| | | EP 4224580 A1 | 09-08-2023 |
| | | JP WO2022070951 A1 | 07-04-2022 |
| | | KR 20230049653 A | 13-04-2023 |
| | | US 2023261198 A1 | 17-08-2023 |
| | | WO 2022070951 A1 | 07-04-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220068034